# EUROPÄISCHE PATENTSCHRIFT

(11) **EP 3 353 797 B1**
(45) Veröffentlichungstag und Bekanntmachung des Hinweises auf die Patenterteilung: **28.08.2019**
(21) Anmeldenummer: 16775119.7
(22) Anmeldetag: 21.09.2016
(51) Int. Cl.: H01F 7/02, A61B 5/05

(54) **MAGNETFELDERZEUGENDE VORRICHTUNG FÜR DAS MAGNETIC PARTICLE IMAGING**
MAGNETIC FIELD-GENERATING DEVICE FOR MAGNETIC PARTICLE IMAGING
DISPOSITIF GÉNÉRATEUR DE CHAMP MAGNÉTIQUE POUR L'IMAGERIE À PARTICULES MAGNÉTIQUES

(30) Priorität: 21.09.2015 DE 102015218122
(43) Veröffentlichungstag der Anmeldung: 01.08.2018
(73) Patentinhaber: Universität zu Lübeck, 23538 Lübeck (DE)
(72) Erfinder: BUZUG, Thorsten, 23627 Groß Sarau (DE); WEBER, Matthias, 94607 Oakland, CA (US)
(74) Vertreter: RCD Patent Giesen, Schmelcher & Griebel Patentwanwälte PartG mbB
(86) Internationale Anmeldenummer: PCT/EP2016/072342
(87) Internationale Veröffentlichungsnummer: WO 2017/050789

(56) Entgegenhaltungen:
- US-A- 4 355 236
- US-A- 4 862 128
- KONKLE JUSTIN ET AL: "Development of a field free line magnet for projection MPI", MEDICAL IMAGING 2011: BIOMEDICAL APPLICATIONS IN MOLECULAR, STRUCTURAL, AND FUNCTIONAL IMAGING, SPIE, 1000 20TH ST. BELLINGHAM WA 98225-6705 USA, Bd. 7965, Nr. 1, 3. März 2011 (2011-03-03) , Seiten 1-7, XP060008566, DOI: 10.1117/12.878435 [gefunden am 2011-03-08]
- KNOPP TOBIAS ET AL: "Generation of a static magnetic field-free line using two Maxwell coil pairs", APPLIED PHYSICS LETTERS, A I P PUBLISHING LLC, US, Bd. 97, Nr. 9, 1. September 2010 (2010-09-01), Seiten 92505-92505, XP012138986, ISSN: 0003-6951, DOI: 10.1063/1.3486118 in der Anmeldung erwähnt

## Beschreibung

Die Erfindung betrifft eine magnetfelderzeugende Vorrichtung, die insbesondere Permanentmagnete in einer Halbach-Anordnung aufweist. Das erzeugte Magnetfeld besitzt eine feldfreie Linie mit einem vorbestimmten Magnetfeldgradienten in einer vorbestimmten Ebene, die als Scanebene für das Magnetic Particle Imaging dienen kann.

Beim Magnetic Particle Imaging (MPI) werden lokale Konzentrationen magnetisierbarer Nanopartikel in einer a priori unbekannten räumlichen Verteilung im Innern eines Patienten ermittelt. Diese Nanopartikel sind nicht radioaktiv, und das MPI-Messverfahren bedarf auch sonst keiner ionisierenden Strahlung. Beispielsweise können super-paramagnetische Eisenoxidpartikel durch ein mit vorbestimmter Frequenz periodisch veränderliches Anregungsmagnetfeld, engl. "drive field", periodisch magnetisiert werden, wobei die Magnetisierung der Partikel nichtlinear von der Magnetfeldstärke abhängt. Durch Erfassen und Analysieren des Zeitverhaltens der Partikelmagnetisierung im Verhältnis zur Charakteristik des Anregungsfeldes kann auf die Partikelkonzentration geschlossen werden.

Zur Eingrenzung der MPI-Messung auf kleine Volumina des Patienten wird das Anregungsmagnetfeld mit einem üblich zeitlich konstanten Selektionsmagnetfeld, engl. "selection field", überlagert. Das Selektionsmagnetfeld weist an wenigstens einem vorbestimmten Punkt der Untersuchungsregion eine Nullstelle auf. Ausgehend von diesem so genannten feldfreien Punkt, kurz: FFP, steigt das Selektionsmagnetfeld in alle Richtungen schnell an, so dass magnetisierbare Nanopartikel schon in geringem Abstand zum FFP in die magnetische Sättigung gelangen. Weit vom FFP entfernte Nanopartikel reagieren dann kaum noch auf das Anregungsmagnetfeld und leisten keinen signifikanten Beitrag zum detektierten Signal. Das MPI-Messsignal stammt vielmehr aus der lokalen Umgebung des FFP und gibt über die dort vorhandene, lokale Partikelkonzentration Auskunft.

Alternativ kann statt eines einzelnen FFP auch eine feldfreie Linie, kurz: FFL, beispielsweise mit einer geeigneten Konfiguration magnetfelderzeugender Spulen generiert werden. Die Verwendung einer FFL anstatt eines einzelnen FFP hat das Potenzial, die Datenakquisitionsdauer um eine Größenordnung zu verringern. Allerdings antworten alle magnetischen Partikel eines im Innern der Spulenanordnung platzierten Objekts entlang der FFL simultan auf eine Änderung des Anregungsfeldes, so dass die Auswertung der MPI-Messsignale im Hinblick auf lokale Konzentrationen, die man dann einzelnen Voxeln des Objekts zuweisen kann, eine aufwendigere numerische Behandlung erfordert als für nur einen FFP.

Es ist Stand der Technik, die Position eines FFP oder einer FFL innerhalb einer Region, aus der MPI-Messdaten gewonnen werden sollen - dem sogenannten "field of view", FOV -, durch Überlagerung des statischen Selektionsfeldes mit einem zeitveränderlichen homogenen Magnetfeld zu verschieben. Dieses homogene Magnetfeld ist speziell für das MPI mit einer FFL senkrecht zum Verlauf der FFL anzulegen und bestimmt durch seine Feldrichtung auch, entlang welcher Ebene die FFL verschoben werden kann. Dadurch ist die Scanebene des MPI, durch die idealerweise ein Patient senkrecht hindurch bewegt werden soll, festgelegt.

Für die zuvor genannte aufwendigere numerische Behandlung der MPI-Messdaten bei Verwendung einer FFL ist es von Bedeutung, dass die FFL innerhalb der Scanebene um ihren Mittelpunkt verdreht werden kann, d.h. dass die MPI-Signalerfassung vergleichbar zu einer Röntgen-CT-Messung unter einer Mehrzahl von Ausrichtungen der FFL relativ zum ruhenden Patienten erfolgen kann. Das homogene Magnetfeld, welches stets der scannenden Seitwärtsbewegung der FFL in der Scanebene dient, muss deshalb bis heute durch ein Spulenpaar erzeugt werden, dass die Drehbewegung der FFL auch apparativ begleitet, d.h. die Spulenanordnung muss wenigstens in Grenzen rotierbar ausgebildet sein. Dies ist sowohl im Hinblick auf Materialverschleiß als auch auf die Handhabung der Starkstrom führenden Zuleitungen zum Spulenpaar von Nachteil.

Dem Fachmann sind sogenannte Halbach-Anordnungen von Permanentmagneten bekannt. Solche Anordnungen können insbesondere als geometrische Objekte ausgebildet sein, in denen mehrere Permanentmagnete, beispielsweise in Stabform, in vorbestimmter Anordnung eingebettet vorliegen. Gewöhnlich bestehen solche Objekte aus einer nicht-magnetischen Materialmatrix, sind etwa Formkörper aus einem ausgehärteten Polymer wie z.B. Polymethylmethacrylat (PMMA), und umfassen starke Permanentmagnete basierend auf Elementen der Seltenen Erden. Die Objekte besitzen dann selbst permanente Magnetfelder, deren Flußdichten in Teilbereichen ihrer Oberfläche größer als die größte Flußdichte irgendeines der eingebetteten Permanentmagneten sein kann. Zugleich besitzen die eine Halbach-Anordnung umfassenden Objekte auch Teilbereiche ihrer Oberfläche, die praktisch magnetfeldfrei sind.

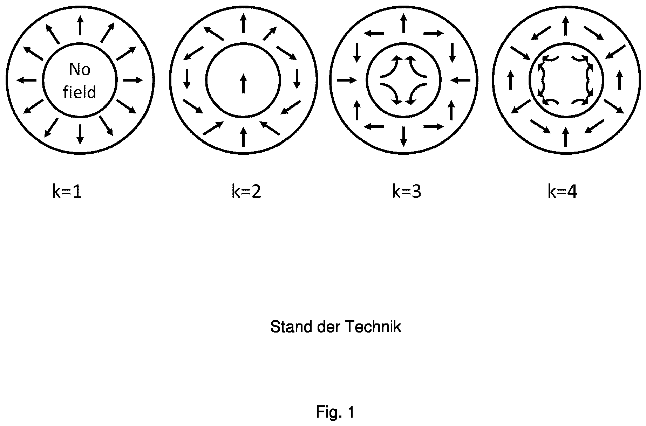

Zu den prominentesten Beispielen für Halbach-Anordnungen umfassende geometrische Objekte zählen die Halbach-Zylinder. Solche Zylinder weisen einen Innen- und einen Außendurchmesser auf und bestehen insofern aus Material, welches einen dicken Zylindermantel, mithin eine Röhre, bildet. Ein Halbach-Zylinder ist dadurch gekennzeichnet, dass die Magnetisierungsrichtung des Mantels entlang des Mantelverlaufs eine stetige Drehung in der Ebene senkrecht zur Zylinderachse aufweist. Da der Mantel geschlossen ist, kehrt die Magnetisierungsrichtung bei einem Umlauf des gesamten Zylindermantels in ihren Ausgangswert zurück. Zur Verdeutlichung wird auf Fig. 1 verwiesen. (Quelle: "Halbach cylinder" by Own work by en:User:Hiltonj - originally uploaded to the English language Wikipedia. Licensed under CC BY-SA 3.0 via Wikimedia Commons - https://commons.wikimedia.org/wiki/File:Halbach cylinder.Pnq#/media/File:Halbach cylinder. png).

Ausgedrückt in ebenen Polarkoordinaten *R, ϕ* ist der - zweidimensionale - Magnetisierungsvektor eines Halbach-Zylindermantels durch *̅M̅*̅= *M*₀ (cos *kϕ,* sin *kϕ*) beschrieben, wobei k eine natürliche Zahl sein muss und oft als Halbach-Ordnung bezeichnet wird.

Fig. 1 zeigt Skizzen der Halbach-Zylinder der ersten vier Ordnungen mit den vorbestimmten Magnetisierungsrichtungen des Mantelmaterials und den sich daraus ergebenden Magnetfeldrichtungen in den - materialfreien - Innenräumen der Zylinder. Offensichtlich ist ein Halbach-Zylinder der Halbach-Ordnung k = 2 von besonderem praktischen Interesse, da er in der Lage ist, ein relativ starkes homogenes Magnetfeld senkrecht zur Zylinderachse in seinem Innern bereitzustellen.

Das Magnetfeld eines idealen Halbach-Zylinders ist im Prinzip exakt analytisch berechenbar. Doch da in der Praxis ohnehin nur Halbach-Zylinder aus einer endlichen Anzahl permanentmagnetischer Segmente erstellt werden und folglich die Verdrehung der Magnetisierungsrichtung im Zylindermantel in diskreten Schritten erfolgt, ist die analytische Berechnung für reale Vorrichtungen kaum handhabbar. Vielmehr verlegt man sich auf die numerische Simulation des Magnetfeldes im Innern des Zylinders. Dabei erkennt man schnell, das die Homogenität des Magnetfeldes für einen Zylinder der Halbach-Ordnung k = 2 für eine reale Anordnung natürlich nur eine Näherung und für den Nahbereich der Zylinderdeckflächen dabei noch nicht einmal eine gute ist. Die Zylinderhöhe darf grundsätzlich nicht zu klein gewählt werden, wenn man die postulierte Geometrie des Magnetfeldes zumindest in Teilbereichen des Zylinderinnern erreichen will.

Gewöhnlich ist die Zylinderhöhe groß gegen den Innendurchmesser des Zylinders, oftmals auch größer als der Außendurchmesser. Zu den bekannten Anwendungen von Halbach-Zylindern der Halbach-Ordnung k = 3 zählt beispielsweise die Lenkung und Fokussierung geladener Partikelstrahlen.

Insbesondere erwähnt Knopp et al. "Generation of a static magnetic field-free line using two Maxwell coil pairs", APPLIED PHYSICS LETTERS 97, 092505, 2010 die stromlose Erzeugung einer FFL im Quadrupolfeld eines Halbach-Zylinders der Halbach-Ordnung k = 3 als eine Möglichkeit für das MPI. Jedoch liegt diese FFL zunächst auf der Symmetrieachse des Halbach-Zylinders, entlang der z.B. ein Patient eingeschoben werden müßte. Die FFL ist insofern zunächst um 90° in eine angemessene Scanebene zu drehen, und dann müssen noch zwei Spulenpaare zum Verschieben der FFL in der Scanebene angeordnet werden. Die Arbeit von Knopp et al. umgeht dies durch Ersetzen des Halbach-Zylinders durch zwei Paare von Maxwellspulen, wobei der Patient nun entlang einer Achse durch eines der Spulenpaare hindurch bewegt werden kann.

Halbach-Zylinder der Halbach-Ordnung k = 2 finden als stromlose Erzeuger homogener Magnetfelder auch bei der Magnetresonanztomographie - MRT - bereits Anwendung, wie die Druckschrift US 2015/177343 A1 erkennen lässt. Es soll hier nur betont werden, dass das field of view bei der genannten Arbeit im Zylinderinnern liegt.

Es existieren weiterhin Anwendungen von Halbach-Zylindern in Magnetgetrieben. Dabei werden mehrere Halbach-Zylinder - üblich unterschiedlicher Halbach-Ordnungen - konzentrisch ineinander gesetzt angeordnet. Eine Drehbewegung eines der Zylinder wird durch die magnetische Wechselwirkung auf den anderen kontaktlos übertragen. Bei den Magnetgetrieben ist die Zylinderhöhe teilweise recht gering, oftmals deutlich kleiner als der Außendurchmesser.

Die Druckschrift EP 1876462 A1 verwendet mehrere Stapel von Halbach-Ringen, die konzentrisch ineinander gesetzt und gegeneinander drehbar sind, um im Innern der konzentrischen Stapel eine möglichst präzise Feinabstimmung des Magnetfeldes zu ermöglichen.

Die vorliegende Beschreibung benutzt für Halbach-Zylinder, deren Höhe kleiner als der Außendurchmesser ausgebildet ist, im Folgenden die Bezeichnung "Halbach-Ring". Die Zylinderhöhe wird im Weiteren für Halbach-Ringe auch als "Ringbreite" bezeichnet.

Zudem wird nachfolgend einem Halbach-Ring der Halbach-Ordnung k = 2 eine Magnetisierungsrichtung zugeschrieben, die mit der Richtung des Magnetfeldes im Innern des Halbach-Rings übereinstimmt. Die Magnetisierungsrichtung eines solchen Halbach-Rings steht insbesondere senkrecht auf der Symmetrieachse des Rings. Die Symmetrieachse des Rings wird hier auch als "Ringachse" bezeichnet. Der Mittelpunkt eines Halbach-Rings ist der geometrische Mittelpunkt. Er liegt im Innern eines Rings auf der Ringachse und fällt mit dem Schwerpunkt zusammen, wenn der Ring z.B. aus einem Material mit homogener Massendichte gebildet ist.

Die Erfindung stellt sich nun die Aufgabe, eine magnetfelderzeugende Vorrichtung für das MPI vorzuschlagen, die in einfacher und energiesparender Weise ein Selektionsmagnetfeld mit einer FFL bereitstellt, wobei die FFL überdies ohne Winkelbeschränkung in einer Scanebene verschiebbar und rotierbar ist.

Die Aufgabe wird gelöst durch eine magnetfelderzeugende Vorrichtung nach Anspruch 1.

Aus US 4862128 A1 ist bekannt, derartige Anordnungen von Halbach-Ringen zum Ablenken von Elektronenstrahlen zu verwenden.

Der Mittelpunktabstand ist der geometrische Abstand der Mittelpunkte beider Ringe.

Die einfachste und zugleich bevorzugte Art der symmetrischen Anordnung der Halbach-Ringe besteht darin, die Ringe koaxial anzuordnen, d.h. dass ihre Ringachsen auf derselben Geraden liegen.

Würde man die Ringe dabei unmittelbar aufeinander legen, so dass die Ringe zusammen einen neuen Ring der doppelten Ringbreite eines Einzelrings bildeten, so wäre der Mittelpunktabstand gerade genau diese Ringbreite. Dies reicht nicht aus. Der Abstand muss größer sein, so dass die beiden Halbach-Ringe eine Anordnung mit einem Luftspalt bilden. In der Mitte dieses Luftspalts liegt die Symmetrieebene der Anordnung.

Sofern die Halbach-Ringe Innendurchmesser größer als zwei Ringbreiten aufweisen - in anderen Worten: die Ringbreiten kleiner als die Innenradien ausgebildet sind -, dann ist es eine bevorzugte Ausgestaltung der Erfindung, wenn die Halbach-Ringe mit einem Mittelpunktabstand wenigsten größer als der halbe Innendurchmesser angeordnet sind.

Die koaxial angeordneten Halbach-Ringe sind um ihre gemeinsame Achse gegeneinander um 180° verdreht, so dass ihre Magnetisierungsrichtungen in entgegengesetzte Richtungen zeigen.

Macht man die soweit beschriebene Anordnung zum Gegenstand einer numerischen Modellierung des erzeugten Magnetfeldes und untersucht man dabei insbesondere die Symmetrieebene, so findet man das Folgende:
1. In der Symmetrieebene liegt eine feldfreie Linie vor. Diese FFL ist in guter Näherung eine Gerade.
2. Außerhalb der FFL in der Symmetrieebene steigt das Magnetfeld dem Betrage nach an mit einem Feldgradienten, der Anwendungen in der MPI erlaubt, wenn die Halbach-Ringe mit entsprechend starken Magneten ausgestattet sind.
3. Über einen ausgedehnten Bereich der Symmetrieebene weist das erzeugte Magnetfeld nur Feldkomponenten entlang der gemeinsamen Achse der Halbach-Ringe auf, also senkrecht zur Symmetrieebene.
4. Legt man ein zusätzliches homogenes Magnetfeld entlang der Halbach-Ringachsen an, das zeitlich veränderlich ist, so kann die FFL dadurch in der Symmetrieebene verschoben werden.

Überdies ist dem Fachmann sofort klar, dass sich die Richtung der FFL zusammen mit den beiden Halbach-Ringen drehen lässt. Es ist daher eine besonders vorteilhafte Ausgestaltung der Vorrichtung, wenn die Halbach-Ringe um ihre Ringachsen drehbar gelagert sind. Sie sollten sich dabei stets simultan und in gleicher Weise drehen, um die Magnetfeldeigenschaften in der Symmetrieebene - die hiernach als Scanebene für das MPI dienen kann - zu bewahren. Vorzugsweise umfasst die Vorrichtung daher auch ein mechanisches Getriebe zur Übertragung der Drehung eines Halbach-Ringes auf den anderen unter Beibehaltung der entgegengesetzten Magnetisierungsrichtungen der Halbach-Ringe.

Eine elektrische Spulenanordnung, die ein homogenes Magnetfeld zur Verschiebung der FFL bereitstellt, muss hingegen zu keiner Zeit bewegt oder gedreht werden, denn das Magnetfeld ist hier parallel zur Drehachse anzulegen. Die Vorrichtung wird somit erfindungsgemäß erweitert um eine Spulenanordnung, besonders bevorzugt ein Helmholtz-Spulenpaar, ausgebildet zur Erzeugung eines homogenen magnetischen Feldes mit Feldrichtung senkrecht zur Symmetrieebene der Halbach-Ring-Anordnung wenigstens in einem Teilbereich der Symmetrieebene.

Weiterhin kann es von Vorteil sein, wenn man die Vorrichtung nicht mit exakt koaxialen Halbach-Ringen aufbaut, sondern ein Verkippen der Halbach-Ringe in Betracht zieht. Die Verkippung muss dabei so erfolgen, dass die Anordnung der Ringe stets symmetrisch zu einer Symmetrieebene bleibt. Beispielsweise können die beiden Halbach-Ringe jeweils um den gleichen Kippwinkel aufeinander zu geneigt werden. So entsteht bevorzugt eine symmetrische Anordnung der Halbach-Ringe, die einen vorbestimmten Kippwinkel der Ringachsen größer als null gegen die Normale der Symmetrieebene aufweist. Der Kippwinkel soll dem Betrage nach bevorzugt aus dem Bereich 5° bis 15° gewählt sein.

Auch in der verkippten Anordnung können die Halbach-Ringe um ihre Ringachsen gedreht werden, wobei erneut beide simultan und in gleicher Weise gedreht werden sollen. Zwar liegen die Magnetisierungsrichtungen der Halbach-Ringe aufgrund der Verkippung nicht mehr so, dass man sie exakt zueinander entgegengesetzt ausrichten kann, aber dies ist auch nicht entscheidend. Wesentlich ist vielmehr, dass die Projektionen der Magnetisierungsrichtungen auf die Symmetrieebene in entgegengesetzte Richtungen zeigen, so dass sich ebendort die für das MPI geeigneten Feldverhältnisse einstellen.

Das Magnetfeld einer solchen Anordnung der Halbach-Ringe kann der Fachmann ohne Mühe modellieren. Dabei zeigt sich:
1. In der Symmetrieebene existiert immer noch eine FFL, die allerdings nur noch ungefähr als Gerade klassifiziert werden kann. Sie liegt aber im Vergleich zur FFL bei koaxialen Ringen in Richtung auf die größte Annäherung der verkippten Halbach-Ringe verschoben vor.
2. Der Feldgradient in der Umgebung der FFL ist nicht mehr so gleichmäßig wie im Fall der koaxialen Ringe, aber dem Betrage nach immer noch ausreichend für MPI.
3. Nach wie vor hat das Magnetfeld über einen großen Bereich der Symmetrieebene in guter Näherung nur Feldkomponenten senkrecht zur Symmetrieebene.
4. Das homogene Magnetfeld einer Spulenanordnung - die Spulen sollen nicht mit den Halbach-Ringen verkippt werden - ist nach wie vor geeignet, die FFL in der Scanebene zu verschieben.

Der erkennbare Vorteil der verkippten Anordnung liegt in der stromlosen Repositionierung der FFL in der Scanebene, mithin in der einstellbaren Lage der FFL ohne das Feld der Spulen.

Wenn man einen Patienten - oder ein Körperteil eines Patienten - durch die Scanebene und somit auch durch die Innenräume der Halbach-Ringe hindurch bewegt, dann muss die FFL letzten Endes in eine region of interest (ROI) im Patienten verschoben werden, in der der MPI-Scan erfolgen soll, wenn man nicht den gesamten Patienten scannen will. Man kann dies durch eine geeignet gewählte Grundbestromung der Spulen erreichen, die etwa die FFL aus ihrer zentralen Lage im Fall der koaxialen Halbach-Ringe auslenkt. Aber der Bestromung der Spulen sind Grenzen gesetzt, z.T. aus technischen Gründen und z.T. aus medizinischen. Denn für den MPI-Scan ist die Lage der FFL in der ROI typisch mit Kilohertz-Frequenz zu variieren, und je höher die von den Spulen erzeugt Magnetfeldstärke dabei sein muss, desto wahrscheinlicher ist das Auftreten unerwünschter physiologischer Effekte, z.B. einer peripheren Nervenstimulation (PNS).

Das Verkippen der Halbach-Ringe schafft für die Positionierung der FFL einen neuen Freiheitsgrad, nämlich den Kippwinkel. Das Magnetfeld ist dabei zunächst zeitlich konstant, und die FFL wird sozusagen von einer neuen "Ruhelage" aus beim MPI-Scan verschoben, was im Mindesten auch den Energieverbrauch der Spulen reduzieren sollte.

Bei der gemeinsamen Verdrehung der verkippten Halbach-Ringe wird die FFL aber nicht allein in der Scanebene rotiert, sondern zugleich auch translatiert und ggf. auch etwas deformiert. Für die MPI-Auswertung heißt dies, dass sie schlicht komplizierter und vermutlich numerisch deutlich aufwendiger wird. Bis heute existiert keine solche Auswertung, und sie ist auch nicht Gegenstand dieser Erfindung.

Aber die Magnetfelder der erfindungsgemäßen Vorrichtung sind für jede konkrete Ausgestaltung der Halbach-Ringe vom Fachmann mit der entsprechenden - kommerziell erhältlichen - Modellierungs-Software berechenbar. Er kann ohne gedankliche Mühe, nämlich nach dem Trial-and-Error Prinzip, die Abstände der Halbach-Ringe variieren, um ein für seine Zwecke z.B. möglichst gleichmäßiges, möglichst lineares Gradientenfeld in der Symmetrieebene mit einer möglichst geraden FFL zu erzeugen. Er kann auch den Verlauf der FFL für jeden verdrehten und/oder verkippten Zustand der Halbach-Ringe mit hoher Genauigkeit berechnen und für die spätere Verarbeitung aufzeichnen. Insofern steht die prinzipielle Ausführbarkeit einer MPI-Messung mit der Erfindung auch bei verkippten Ringen nicht in Frage.

Für die Vorrichtung mit koaxial angeordneten Halbach-Ringen ist die MPI-Auswertung aus dem bekannten Stand der Technik ohne weiteres adaptierbar. Die Erfindung eignet sich deshalb vorzugsweise zur Erzeugung eines Selektionsfeldes für das Magnetic Particle Imaging.

Zur Verdeutlichung der Erfindung anhand eines Ausführungsbeispiels dienen die nachfolgenden Erläuterungen und Figuren. Dabei zeigt:
Fig. 2 die Aufsichtskizze (xy-Ebene) einer ringförmigen Anordnung aus diskreten quaderförmigen Permanentmagneten angeordnet in drei konzentrischen Kreisen;
Fig. 3 die Seitensichtskizze (yz-Ebene) der Anordnung zweier Halbach-Ringe nach der Lehre der Erfindung mit Darstellung des Magnetfeldes im Luftspalt;
Fig. 4 eine Skizze der Magnetfeldkomponenten in der Symmetrieebene (xy-Ebene) der Anordnung der Halbach-Ringe.

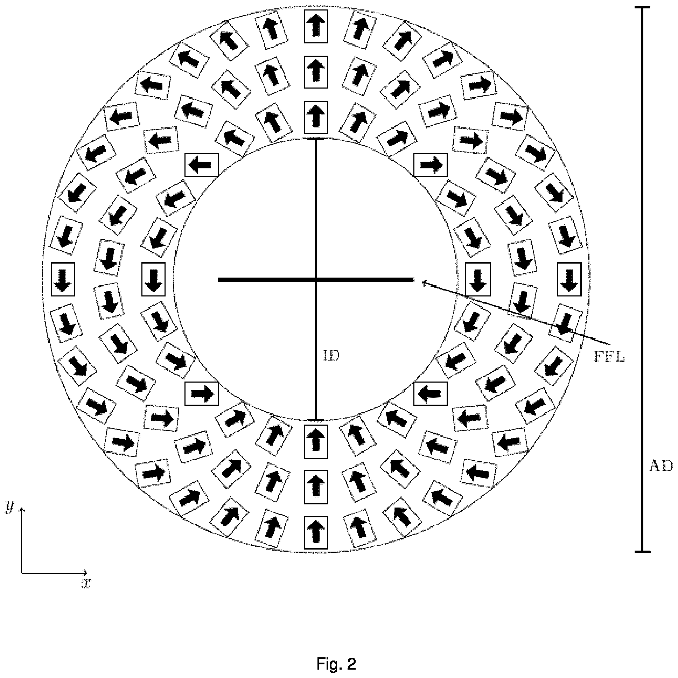

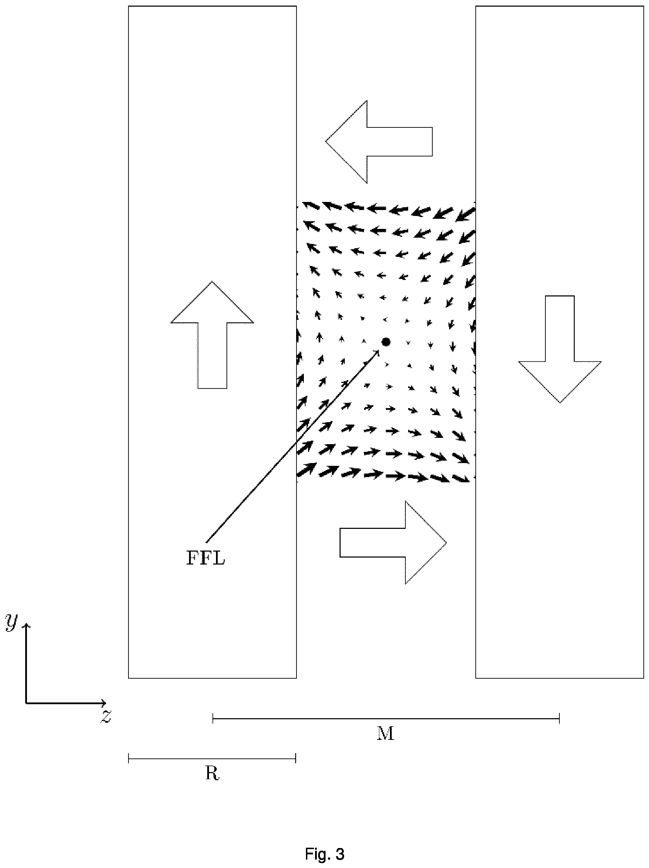

Die in den Fig. 2 und 3 gezeigte Vorrichtung ist so dimensioniert, dass sie MPI-Messungen an Kleintieren, z.B. an Mäusen, gestattet.

Fig. 2 zeigt den Aufbau eines der Halbach-Ringe, der aus quaderförmigen Permanentmagneten - bestehend aus Neodym-Eisen-Bor mit einer Remanenz von 1,42 Tesla und Magnetisierungseigenschaft N52 - in einer nicht-magnetischen Kunststoffmatrix zusammengesetzt ist. Die Kunststoffmatrix wird hier mittels eines 3D-Druckers hergestellt. Fast alle druckbaren, aushärtenden Polymere sind als Kunststoffmatrix geeignet. Die Quader haben eine Größe von 42 mm x 10 mm x 7.2 mm und sind entlang der 10 mm-Seite magnetisiert. Der Halbach-Ring umfasst 90 Einzelmagnete, wobei sich die Magnete auf drei Ringe mit (von innen nach außen) jeweils 24, 30 und 36 Magneten aufteilen. Die Magnete sind auf Kreisen mit den Radien 49.6 mm, 63.6 mm und 77.6 mm angeordnet. Die Pfeile stellen dabei die lokale Magnetisierungsrichtung dar. Der Halbach-Ring weist einen Innendurchmesser ID von 87 mm, einen Außendurchmesser AD von 167,6 mm und eine Ringbreite R von 42 mm auf. Die Magnetisierungsrichtung des Halbach-Rings weist exakt in y-Richtung, während die Ringachse in die Zeichenebene hinein, in z-Richtung, verläuft.

Fig. 2 zeigt auch die Lage der feldfreien Linie FFL in der erfindungsgemäßen Anordnung. Wichtig: Die gezeigte FFL ist *kein* Erzeugnis des in Fig. 2 dargestellten *einen* Halbach-Rings. Sie entsteht erst in der Anordnung der Fig. 3.

In Fig. 3 sind zwei Halbach-Ringe gemäß Fig. 2 in der Seitenansicht (yz-Ebene) zu sehen, wobei die Magnetisierungsrichtungen der Ringe bzw. die Projektionen der Magnetisierungsrichtungen auf die Symmetrieebene der Anordnung in entgegengesetzte Richtungen zeigen. Die aus der Anordnung resultierenden Feldkomponenten löschen sich genau im Zentrum aus, sodass dort die feldfreie Linie FFL entsteht. Es ist hier hervorzuheben, dass der Mittelpunktabstand M der Halbach-Ringe mit 87 mm nicht nur größer ist als die Ringbreite R oder als der halbe Innendurchmesser ID, sondern im Beispiel sogar genau dem Innendurchmesser ID der Ringe entspricht.

Dabei ist zu verstehen, dass der Mittelpunktabstand M ein Optimierungsparameter ist, den sich der Fachmann in Anbetracht seiner konkreten Halbach-Ringe einmal selbst anhand einer numerischen Modellierung für verschiedene Abstände der Ringe ermitteln muss. Dies wird ihm als einmalige Voruntersuchung mit handelsüblicher Software nicht schwer fallen. Er kann dabei das resultierende Feld nach seinen eigenen Optimalitätskriterien bewerten.

Im vorliegenden Beispiel wurden die Geradlinigkeit der FFL und die Gleichmäßigkeit des Magnetfeldgradienten in der Nähe der FFL optimiert. Dass die Modellierung zur Optimierung hier ergibt, dass der Mittelpunktabstand M gerade dem Innendurchmesser ID entsprechen soll, kann nicht ohne weiteres als allgemeine Regel aufgefasst werden. Aber es lässt sich als Daumenregel durchaus empfehlen, die Suche nach dem Mittelpunktabstand M der Halbach-Ringe für ein brauchbares MPI-Selektionsfeld auch dann im Bereich des Innendurchmessers ID der Ringe zu beginnen, wenn die Ringbreite R deutlich kleiner als der Innendurchmesser ID, oder wie im Beispiel sogar kleiner als der halbe Innendurchmesser ID, ist.

Wie Fig. 3 weiter darstellt, sind genau in der Symmetrieebene nur Feldkomponenten in z-Richtung, d.h. entlang der Ringachsen, vorhanden. Die großen Pfeile deuten die lokale Hauptmagnetfeldrichtung an, wobei die in den Ringen gezeichneten Pfeile zugleich den Magnetisierungsrichtungen der Halbach-Ringe entsprechen. Die kleinen Pfeile im Luftspalt zwischen den Ringen beschreiben die lokale Magnetfeldrichtung und durch ihre Länge die Magnetfeldstärke, die sich mittels einer numerischen Modellierung des Magnetfelds der erfindungsgemäßen Anordnung errechnen lässt.

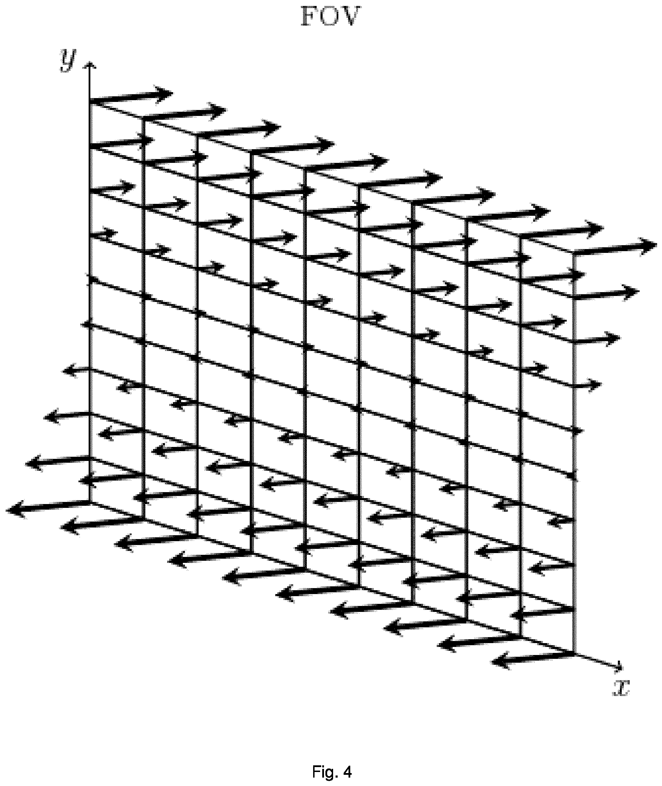

Für die Zwecke des MPI ist das Magnetfeld in der Symmetrieebene der Anordnung, die als Scanebene für die FFL dienen soll, von besonderer Bedeutung. Fig. 4 zeigt das Resultat der numerischen Modellierung genauer für diese Ebene. Es treten nur z-Komponenten des Magnetfeldes über einen ausgedehnten Bereich von etwa 50 mm x 50 mm auf. Dieser Bereich definiert das FOV des MPI, in dem sich die FFL nunmehr durch ein entlang der Ringachsen (z-Achse) gerichtetes, homogenes Magnetfeld verschieben lässt. Dies gilt auch für beliebige simultane und in gleicher Weise vollführte Verdrehungen der beiden Halbach-Ringe.

## Patentansprüche

1. Magnetfelderzeugende Vorrichtung aufweisend zwei baugleiche, aus Permanentmagneten gebildete Halbach-Ringe der Halbach-Ordnung k = 2 mit jeweils vorbestimmtem Innendurchmesser (ID) und Außendurchmesser (AD) und vorbestimmter Ringbreite (R) und Magnetisierungsrichtung, wobei die Halbach-Ringe mit einem Mittelpunktabstand (M) größer als die Ringbreite (R) und kleiner als der Außendurchmesser (AD) symmetrisch zu einer in der Mitte eines Luftspaltes liegenden Symmetrieebene angeordnet sind, wobei die Projektionen der Magnetisierungsrichtungen auf die Symmetrieebene in entgegengesetzte Richtungen zeigen, **gekennzeichnet durch** eine Spulenanordnung, bevorzugt ein Helmholtz-Spulenpaar, ausgebildet zur Erzeugung eines homogenen magnetischen Feldes mit Feldrichtung senkrecht zur Symmetrieebene der Halbach-Ringe wenigstens in einem Teilbereich der Symmetrieebene.

2. Magnetfelderzeugende Vorrichtung nach Anspruch 1, **dadurch gekennzeichnet, dass** die Halbach-Ringe Innendurchmesser (ID) größer als zwei Ringbreiten (R) aufweisen und mit einem Mittelpunktabstand (M) größer als der halbe Innendurchmesser (ID) angeordnet sind.

3. Magnetfelderzeugende Vorrichtung nach Anspruch 1 oder 2, **dadurch kennzeichnet, dass** die Halbach-Ringe koaxial angeordnet sind.

4. Magnetfelderzeugende Vorrichtung nach Anspruch 1 oder 2, **dadurch gekennzeichnet, dass** die symmetrische Anordnung der Halbach-Ringe einen vorbestimmten Kippwinkel der Ringachsen größer als null gegen die Normale der Symmetrieebene aufweist.

5. Magnetfelderzeugende Vorrichtung nach Anspruch 4, **dadurch gekennzeichnet, dass** der Kippwinkel aus dem Intervall 5° bis 15° vorbestimmt ist.

6. Magnetfelderzeugende Vorrichtung nach Anspruch 5, **gekennzeichnet durch** eine Einrichtung ausgebildet zur zeitlich periodischen Bestromung der Spulenanordnung.

7. Magnetfelderzeugende Vorrichtung nach einem der vorangehenden Ansprüche, **dadurch gekennzeichnet, dass** die Halbach-Ringe um ihre Ringachsen drehbar gelagert sind.

8. Magnetfelderzeugende Vorrichtung nach Anspruch 7, **gekennzeichnet durch** ein mechanisches Getriebe zur Übertragung der Drehung eines Halbach-Ringes auf den anderen unter Beibehaltung der entgegengesetzten Magnetisierungsrichtungen der Halbach-Ringe.

9. Verwendung einer Vorrichtung nach einem der vorangehenden Ansprüche zur Erzeugung eines Selektionsfeldes für das Magnetic Particle Imaging.

## Claims

1. A magnetic-field generating device exhibiting two structurally identical Halbach rings, formed from permanent magnets, of the Halbach order k = 2 having in each case a predetermined inside diameter (ID) and outside diameter (AD) and predetermined ring width (R) and magnetization direction, whereby the Halbach rings having a center-point distance (M) greater than the ring width (R) and smaller than the outside diameter (AD) are arranged symmetrically relative to a plane of symmetry located in the center of an air gap, the projections of the magnetization directions onto the plane of symmetry pointing in opposite directions **characterized in** a coil arrangement, preferably a Helmholtz-Coil-Pair, construed for generation of a homogeneous magnetic field having a field direction perpendicularly to the plane of symmetry of the Halbach rings at least in a portion of the plane of symmetry.

2. The magnetic-field generating device according to Claim 1, **characterized in that** the Halbach rings exhibit inside diameters (ID) greater than two ring widths (R) and are arranged at a center-point distance (M) greater than half the inside diameter (ID).

3. The magnetic-field generating device according to Claim 1 or 2, **characterized in that** the Halbach rings are arranged coaxially.

4. The magnetic-field generating device according to Claim 1 or 2, **characterized in that** the symmetric arrangement of the Halbach rings exhibits a predetermined tilting angle of the ring axes greater than zero relative to the normal of the plane of symmetry.

5. The magnetic-field generating device according to Claim 4, **characterized in that** the tilting angle is predetermined from the interval 5° to 15°.

6. The magnetic-field generating device according to Claim 5, **characterized by** a unit designed for temporally periodically energizing the coil arrangement.

7. The magnetic-field generating device according to one of the preceding claims, **characterized in that** the Halbach rings are mounted so as to be rotatable about their ring axes.

8. The magnetic-field generating device according to Claim 7, **characterized by** a mechanical gear for transmitting the rotation of one Halbach ring to the other while maintaining the opposite magnetization directions of the Halbach rings.

9. Use of a device according to one of the preceding claims for generating a selection field for Magnetic Particle Imaging.

## Revendications

1. Dispositif produisant un champ magnétique présentant deux anneaux de Halbach de configuration identique, constitués d'aimants permanents de l'ordre de Halbach k=2 avec respectivement un diamètre intérieur (ID) et un diamètre extérieur (AD) prédéterminé et une largeur d'anneau et une direction de magnétisation prédéterminée (R), dans lequel les anneaux de Halbach sont disposés avec une distance par rapport au point central (M) plus grande que la largeur d'anneau (R) et plus petite que le diamètre extérieur (AD) symétriquement à un plan de symétrie situé au centre d'un entrefer, dans lequel les projections des directions de magnétisation sont dirigées sur le plan de symétrie dans des directions opposées, **caractérisé par** un dispositif de bobine, de préférence une paire de bobines de Helmholtz, configurée afin de produire un champ magnétique homogène avec une direction de champ perpendiculaire au plan de symétrie des anneaux de Halbach au moins dans une zone partielle du plan de symétrie.

2. Dispositif produisant un champ magnétique selon la revendication 1, **caractérisé en ce que** les anneaux de Halbach présentent un diamètre intérieur (ID) plus grand que deux largeurs d'anneau (R) et sont disposés avec une distance par rapport au point central (M) plus grande que la moitié du diamètre intérieur (ID).

3. Dispositif produisant un champ magnétique selon la revendication 1 ou 2, **caractérisé en ce que** les anneaux de Halbach sont disposés coaxialement.

4. Dispositif produisant un champ magnétique selon la revendication 1 ou 2, **caractérisé en ce que** la disposition symétrique des anneaux de Halbach présente un angle de basculement des axes annulaires plus grand que zéro par rapport à la normale du plan de symétrie.

5. Dispositif produisant un champ magnétique selon la revendication 4, **caractérisé en ce que** l'angle de basculement est prédéterminé d'après l'intervalle de 5° à 15°.

6. Dispositif produisant un champ magnétique selon la revendication 5, **caractérisé par** un dispositif configuré pour l'alimentation en courant périodique temporaire du dispositif de bobine.

7. Dispositif produisant un champ magnétique selon une des revendications précédentes, **caractérisé en ce que** les anneaux de Halbach sont positionnés rotativement autour de leurs axes rotatifs.

8. Dispositif produisant un champ magnétique selon la revendication 7, **caractérisé par** une transmission mécanique pour la transmission de la rotation d'un anneau de Halbach sur l'autre en maintenant les directions de magnétisation opposées des anneaux de Halbach.

9. Utilisation d'un dispositif selon une des revendications précédentes pour la production d'un champ de sélection pour l'imagerie par particules magnétiques.
